# EUROPEAN PATENT SPECIFICATION

(11) **EP 3 522 921 B1**
(45) Date of publication and mention of the grant of the patent: **23.12.2020**
(21) Application number: 17781085.0
(22) Date of filing: 05.10.2017
(51) Int. Cl.: A61K 39/35, A61K 39/36, C07K 14/435, A61K 38/02, C07K 1/00, A61P 37/08, C07K 14/415, A61K 39/00

(54) **PREVENTION OF ALLERGY**
PRÄVENTION VON ALLERGIEN
PRÉVENTION DE L'ALLERGIE

(30) Priority: 05.10.2016 EP 16192425; 16.05.2017 EP 17171268
(43) Date of publication of application: 14.08.2019
(73) Proprietor: ASIT BioTech S.A., 1200 Bruxelles (BE)
(72) Inventor: LEGON, Thierry, 3370 Roosbeek (BE); THIRION, Gaetan, 5530 Purnode (BE)
(74) Representative: dompatent von Kreisler Selting Werner - Partnerschaft von Patent- und Rechtsanwälten mbB
(86) International application number: PCT/EP2017/075398
(87) International publication number: WO 2018/065538

(56) References cited:
- EP-A1- 1 872 792
- WO-A1-2008/000783
- WO-A1-2009/083589
- WO-A1-2012/172037
- WO-A1-2015/155310
- US-A1- 2016 030 553
- LARCHE MARK ET AL: "Peptide-based therapeutic vaccines for allergic and autoimmune diseases", NATURE MEDICINE, vol. 11, no. 4, April 2005 (2005-04), pages S69-S76, XP002509273, NATURE PUB. CO ISSN: 1078-8956, DOI: 10.1038/NM1226
- FUKUSHIMA YOICHI ET AL: "Preventive effect of whey hydrolysate formulas for mothers and infants against allergy development in infants for the first 2 years", JOURNAL OF NUTRITIONAL SCIENCE AND VITAMINOLOGY, vol. 43, no. 3, 1997, pages 397-411, XP002775545, ISSN: 0301-4800
- JONSDOTTIR SIGRIDUR ET AL: "Developing a preventive immunization approach against insect bite hypersensitivity using recombinant allergens: A pilot study", VETERINARY IMMUNOLOGY AND IMMUNOPATHOLOGY, vol. 166, no. 1-2, July 2015 (2015-07), pages 8-21, XP002775546,
- VICTOR JEFFERSON R ET AL: "Maternal immunization with ovalbumin prevents neonatal allergy development and up-regulates inhibitory receptor Fc gamma RIIB expression on B cells.", BMC IMMUNOLOGY, vol. 11, 11, 11 March 2010 (2010-03-11), pages 1-10, XP002775547, ISSN: 1471-2172, DOI: 10.1186/1471-2172-11-11

## Description

The present invention relates to preparations for preventing the development of an allergy and methods and uses related thereto.

Allergies are based on a hypersensitivity of the immune system to something in the environment that usually causes little or no problem for most people. It comprises hay fever, food allergies, atopic dermatitis, allergic asthma and anaphylaxis. Allergies are common in the developed world.

Common allergens are pollens, house dust mites, moulds, drugs, foods and animal hair and dander.

Allergic diseases have a strong genetic relation. Allergic parents are more likely to have allergic children. It seems that the likelihood of developing allergies is inherited.

The most common allergy diseases are rhinitis, asthma and atopic dermatitis. Allergic asthma is a chronic inflammatory disorder. Symptomatic treatment of allergic disorders comprises administration of antihistamines, β-antagonists and corticosteroids.

Furthermore, the so called "specific" immunotherapy is based on a hyposensitization. Typically, patients with confirmed allergy are administered with subcutaneous injection of the specific offending allergens. Treatment is started with small allergen doses and the doses are increased. Treatment is typically maintained for several years. This type of treatment suffers from poor patient compliance and has been questioned due to safety reasons because a patient can suffer from severe anaphylactic reactions.

In addition to methods comprising repeated subcutaneous injections there are also oral hyposensitization methods.

US 4,822,611 discloses a method for treating existing allergies comprising oral treatment with allergens. It describes the use of commercially available "bulk" allergenic extracts showing batch-to-batch variation and differences in extracts from different manufactures. The preparation of these extracts is not described.

GB 1 247 614 discloses a method of extracting an allergen. The aim of this method is to have a more complete and effective allergenic extract by including all extractable components of the allergen.

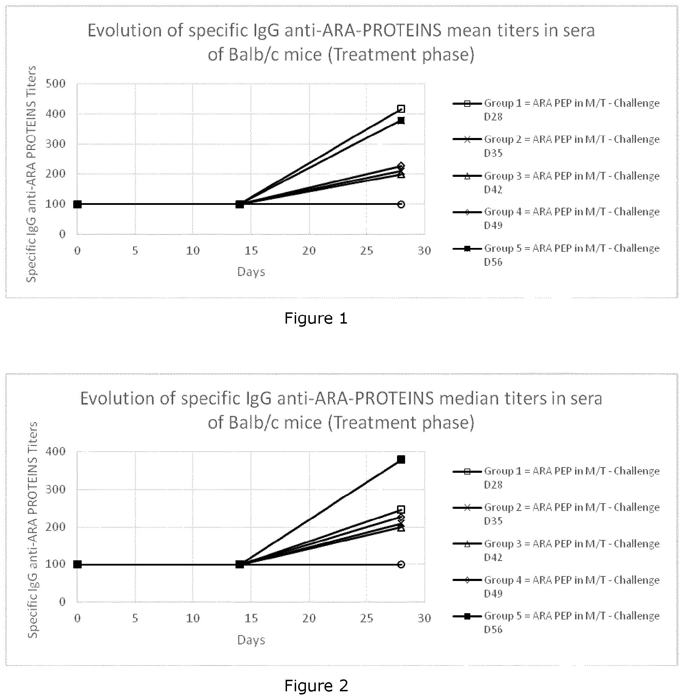

US 5,770,698 discloses a process for purifying extracts of allergenically active proteins. The spectrum of figure 2 of US '698 does not present a peak at 280nm. This implies that the extract contains significant amount on non-protein impurities.

WO 99/22762 discloses a similar method; therefore, the product comprises large amounts of non-protein impurities, too.

US 6,312,711 discloses a pharmaceutically or food composition intended for treating pathologies associated with graft rejection or allergic autoimmune reaction comprising the administration of a complex of a stress protein and epitopes of an antigenic structure.

WO 2013/011095 discloses a pharmaceutical preparation for subcutaneous injection comprising between 0.5 ng and 200 µg of HSP70 between 0.5 and 100 µg of fragments of an antigenic structure.

Fukushima Yoichi et al, Journal of Nutritional Science and Vitaminology, 43 (1997), 397-411 discloses a preventive effect of whey hydrolysate formulas for mothers and infants against allergy development.

Despite strong efforts in the development of treatment methods, it is still not sufficiently addressed. There is still a need for improved treatments of allergies.

Up to now, there has been no prophylactic treatment except avoidance of exposure to allergens.

Surprisingly, it has been found that it is possible to make a prophylactic treatment to avoid development of allergies.

The invention relates to a preparation comprising peptides from a natural allergen for use in the prevention of allergy, wherein the preparation is administered prior to the development of any allergy against the allergen used for the preparation.

The present invention uses peptides of allergens to prevent development of allergy by administering small amounts of the peptides to prevent a later development of an allergy.

According to prior art, a patient suffering from allergy is treated with a drug to supress the symptoms of allergy or by hyposensitization which may reduce the patient reactions to allergens.

In contrast to these methods the present invention is intended to prevent development of the allergy in a patient that is at a risk of developing an allergy.

Therefore, the subject matter of the present invention is a preparation comprising peptides of a natural allergen for use in the prevention of allergy or more specifically for the prevention of development of allergy.

Subject matter of the invention is a preparation comprising peptides from natural allergens for use in the prevention of development of allergy.

In other words, the invention provides a method by which a tolerogenic or positive memory effect can be induced towards a compound that is not yet allergenic but could become allergenic in a patient at risk.

Preferably the preparation is administered 2 to 10 times, preferably at intervals of 2 to 10 days, for example once per week.

The preparation may be administered in constant amounts or increasing amounts.

Preferably, the preparation is free of immune stimulating adjuvants.

For example, if one or both of the parents are suffering from peanut allergy there is an increased risk that the children develop peanut allergy as well. Peanut allergy may be life threatening.

According to the method of the invention, a child could be treated prior to any contact with peanut to develop a status of the immune system that prevents development of the allergy to peanut.

The peptides of an allergen are hydrolyzed allergen peptides.

In one embodiment, said hydrolyzed allergen peptides are obtainable by
a) extracting a natural source of allergens comprising allergenic proteins to form an extract,
b) purifying of said extract to remove non-protein components to form a purified extract,
c) denaturing said purified extract to form a purified denatured extract,
d) hydrolysing the purified denatured extract to form hydrolysed allergen peptides.

In a further embodiment, said hydrolyzed allergen peptides are obtainable by
a) extracting a source of allergens comprising allergenic proteins to form an extract,
b) purifying the extract to remove non-protein components to form a purified extract,
c) denaturing the purified extract with a first denaturing agent to form a purified denatured extract,
d) refining the purified denatured extract to remove impurities to form a refined denatured extract,
e) denaturing the refined denatured extract with a second denaturing agent to form denatured allergen mixture, and
f) hydrolyzing the denatured allergen mixture to form the hydrolyzed allergen peptides.

Preferred allergens are selected among pollen allergens, milk allergens, venom allergens, egg allergens, weed allergens, grass allergens, tree allergens, shrub allergens, flower allergens, vegetable allergens, grain allergens, fungi allergens, fruit allergens, berry allergens, nut allergens, seed allergens, bean allergens, fish allergens, shellfish allergens, seafood allergens, meat allergens, spices allergens, insect allergens, mite allergens, mould allergens, animal allergens, pigeon tick allergens, worm allergens, soft coral allergens, animal dander allergens, nematode allergens, allergens of Hevea brasiliensis.

Preferably, natural allergens are used and hydrolyzed, although recombinant preparations might be suitable as well.

In one embodiment, denaturing is performed with a denaturing agent selected from the group of chaotropic agents, reducing agents and mixtures thereof, preferably among urea, guanidinium chloride, dithiotreitol, thioglycerol, β-mercaptoethanol, TCEP (tris(2-carboxyethyl)phosphine) and mixtures thereof.

In one embodiment, the hydrolysis is performed with an enzyme, preferably pepsin, trypsin or chymotrypsin, more preferably wherein hydrolyzing is performed in the presence of a chaotropic agent, preferably selected from urea and guanidinium chloride and reducing reagent preferably from TCEP or DTT.

In one embodiment, the method further comprises purifying the hydrolyzed allergens to remove peptides with molecular weights above 10.000 Da and below 1.000 Da, wherein 70%, more preferably 80% of the peptides are between 10.000 Da and 1.000 Da.

A further embodiment is the use of the preparation as an allergy vaccine.

A further embodiment is a method of preventing allergy comprising administering the preparation of the invention to a patient at risk of developing allergy.

WO 2008/000783 describes a method of purifying allergens overcoming at least some of the drawbacks of prior art, especially to provide antigens from natural allergens with a significant reduced capability to trigger allergenicity reaction compared to the crude allergen extract but able to stimulate T-cells as well.

WO 2012/172037 discloses a method for the production of hydrolyzed allergens.

The effects of the present invention are exemplified as follow:

### Example 1 - Peanut

### 1. AIM OF THE STUDY:

Evaluation of the induction of a possible "memory effect" in Balb/c mice treated twice per week during 3 weeks with 400µg of peanuts peptides (ARA/PEP_SOL UBT13J03) in mannitol-trehalose environment (the injections were performed sub-cutaneously without any adjuvant) and challenged by a unique intraperitoneal (ip) injection of 100µg of peanut native proteins without any adjuvant.

### 2. Summary of the protocol:

| | Group 1 | Group 2 | Group 3 | Group 4 | Group 5 | Group 6 |
|---|---|---|---|---|---|---|
| Number of animals | 10 | 10 | 10 | 10 | 10 | 10 |
| Treatment : Antigens in M/T | ARA/PEP_SOL 13J03 | ARA/PEP_SOL 13J03 | ARA/PEP_SOL 13J03 | ARA/PEP_SOL 13J03 | ARA/PEP_SOL 13J03 | PLACEBO |
| Injected dose (treatment) | 400µg | 400µg | 400µg | 400µg | 400µg | 400µg |
| Way of administration (treatment) | SC | SC | SC | SC | SC | SC |
| Injected volume (treatment) | 300µl | 300µl | 300µl | 300µl | 300µl | 300µl |
| Injections calendar (treatment) | J0-4-7-11-14 et 18 | J0-4-7-11-14 et 18 | J0-4-7-11-14 et 18 | J0-4-7-11-14 et 18 | J0-4-7-11-14 et 18 | J0-4-7-11-14 et 18 |
| Challenge | ARA/ALL_SOL NATIVES 13I12 | ARA/ALL_SOL NATIVES 13I12 | ARA/ALL_SOL NATIVES 13I12 | ARA/ALL_SOL NATIVES 13I12 | ARA/ALL_SOL NATIVES 13I12 | ARA/ALL_SOL NATIVES 13I12 |
| Injected dose (challenge) | 100µg | 100µg | 100µg | 100µg | 100µg | 100µg |
| Way of administration (challenge) | IP | IP | IP | IP | IP | IP |
| Injected volume (challenge) | 200µl | 200µl | 200µl | 200µl | 200µl | 200µl |
| Injection calendar (challenge) | J28 | J35 | J42 | J49 | J56 | J28 |

### 3. Denomination of the groups:

| | |
|---|---|
| Group 1 | 13J03-DP / Challenge J28 |
| Group 2 | 13J03-DP / Challenge J35 |
| Group 3 | 13J03-DP / Challenge J42 |
| Group 4 | 13J03-DP / Challenge J49 |
| Group 5 | 13J03-DP / Challenge J56 |
| Group 6 | PLACEBO (M/T) / Challenge J28 |

### 4. Correspondence between Days post-beginning of the treatment and Days post-Challenge:

All the groups have the same calendar for the treatment phase till the Day 28

| **Groups** | **Days post-beginning of the treatment** | **Days Post-Challenge** |
|---|---|---|
| **1 and 6** | **28-35-42-49-56-63-70** | **0-7-14-21-28-35-42** |
| **2** | **35-42-49-56-63-70** | **0-7-14-21-28-35** |
| **3** | **42-49-56-63-70** | **0-7-14-21-28** |
| **4** | **49-56-63-70** | **0-7-14-21** |
| **5** | **56-63-70** | **0-7-14** |

### Caution:

ARA/PEP_SOL is commonly used for peanut peptides.
ARA/ALL_SOL is commonly used for peanut proteins

### 5. Results:

Here are the results obtained by ELISA for the production of specific IgG anti-ARA proteins **(native** - **native proteins coating)**

### a. Evolution of IgG production BEFORE any challenge (from Day 0 to Dav 28):

Figures 1 and 2 show that the treatment of the mice with peanut peptides as drug product leads to a very weak production of specific IgG anti-ARA PROTEINS till the Day 28. The mice treated with PLACEBO show no specific IgG anti-ARA PROTEINS production.

### b. Evolution of IgG production AFTER the challenge (from the Challenge Day to the Day 42 post-challenge): caution: not all groups reach this Day 42 post-challenge!!

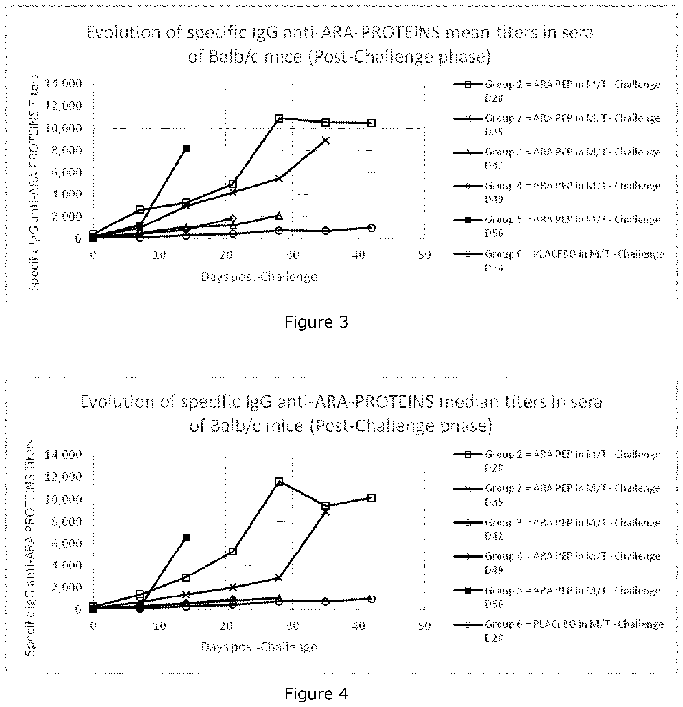

Figures 3 and 4 show the production of specific IgG anti-ARA PROTEINS in the sera of mice belonging to the ARA-peptides treated groups and to the Placebo-group. This production increases with different speed according to the moment when the challenge occurred. When the challenge is performed at Day 28, 35 and 56 post-beginning of the treatment, we can observe that the specific IgG anti-ARA PROTEINS production take off from Placebo-s one 2 weeks after the challenge. For the groups with the challenge occurred at Day 42 and 49 post-beginning of the treatment, no difference in the IgG production is observed to the Placebo's one till Day 21 post-challenge (and even to Day 28 post-Challenge for the D42-Challenge Group = Group 3).

For the group 1, when the Challenge is performed at Day 28 post-beginning of the treatment, a maximum in the specific IgG anti-ARA PROTEINS production seems to be reached at Day 28 post-Challenge after the going down to a plate state at D35 (and later) post-treatment.

For the group 2, when the Challenge is performed at Day 35 post-beginning of the treatment, a maximum is reached at Day 35 post-challenge but we don't have the late time-points so, we may not conclude if this point is well a maximum or if the production will increase again at late time-points.

The higher titers values of each group are the following ones:

| Days Post-Challenge | Specific IgG anti-ARA PROTEINS **MEAN** / **MEDIAN** TITERS |
|---|---|
| | Maximum value Titers |
| Group 1 | D28 = **10.892** / **11.653** |
| Group 2 | D35 = **8.900** / **8.894** |
| Group 3 | D28 = **2.103** / **1.022** |
| Group 4 | D21 = **1.849** / **947** |
| Group 5 | D14 = **8.171** / **6.543** |
| Group 6 | D42 = **990** / **990** |

### 6. Conclusions:

From all these results, we can conclude the following points:
- The treatment with ARA-peptides as described in this protocol induces a very weak production of specific IgG anti-ARA PROTEINS till Day 28 (post-beginning of the treatment)
- No specific IgG production is observed in Placebo groups during the treatment phase till Day 28 (post-beginning of the treatment)
- The challenge with a unique dose of 100µg of ARA native proteins potentializes the effect of the treatment allowing an induction of the production of specific IgG anti-ARA PROTEINS reaching a maximum 4 weeks after the challenge for the group challenged at Day 28 post-beginning of the treatment. This effect is not observed in the Placebo-groups that received also the same challenge. So, we can conclude that the challenge is not sufficient per se to induce the IgG production 4 weeks after the challenge (as observed in Placebo-groups) but allows the expression of the ARA-peptides treatment. Obviously, the ARA-peptides treatment can prime the immune system allowing a "memory effect" after a challenge with native proteins.
- The amplitude of the IgG response depends on the moment separating the end of the treatment and the challenge. The response is higher groups challenged at Day 28, 35 and 56 post-beginning of the treatment. Surprisingly, the groups challenged at Day 42 and 49 post-beginning of the treatment seem not to show a significative difference than the placebo-group. Maybe the production is delayed in these groups... We could answer that question when we will have tested the late time-points.
- All these results are related to ARA native proteins that are the closest proteins to natural allergens.

### Example 2 - House dust mite

Evaluation of the induction of a possible "memory effect" in Balb/c mice treated twice per week during 3 weeks with 400µg of house dust mite peptides (HDM/PEP_SOL UBT15H20) in mannitol-trehalose environment (the injections were performed sub-cutaneously without any adjuvant) and challenged by a unique ip injection of 100µg of house dust mite native proteins without any adjuvant.

### 7. Summary of the protocol:

| | **Group 1** | **Group 2** | **Group 3** | **Group 4** | **Group 5** | **Group 6** | **Group 7** | **Group 8** |
|---|---|---|---|---|---|---|---|---|
| **Number of animals** | **15** | **15** | **15** | **15** | **10** | **10** | **10** | **10** |
| **Treatment : Antigens in M/T** | **HDM/PEP_SOL 15H20** | **HDM/PEP_SOL 15H20** | **HDM/PE P_SOL 15H20** | **HDM/PE P_SOL 15H20** | **PLACEBO** | **PLACEBO** | **PLACEBO** | **PLACEBO** |
| **Injected dose (treatment)** | **400µg** | **400µg** | **400µg** | **400µg** | **400µg** | **400µg** | **400µg** | **400µg** |
| **Way of administration (treatment)** | **SC** | **SC** | **SC** | **SC** | **SC** | **SC** | **SC** | **SC** |
| **Injected volume** (treatment) | **300µl** | **300µl** | **300µl** | **300µl** | **300µl** | **300µl** | **300µl** | **300µl** |
| **Injections calendar (treatment)** | **J0-4-7-11-14 et 18** | **J0-4-7-11-14 et 18** | **J0-4-7-11-14 et 18** | **J0-4-7-11-14 et 18** | **J0-4-7-11-14 et 18** | **J0-4-7-11-14 et 18** | **J0-4-7-11-14 et 18** | **J0-4-7-11-14 et 18** |
| **Challenge** | **HDM/ALL_SOL NATIVES 13G04nat** | **HDM/ALL_SOL NATIVES 13G04nat** | **HDM/ALL _SOL NATIVES 13G04nat** | **HDM/ALL _SOL NATIVES 13G04nat** | **HDM/ALL _SOL NATIVES 13G04nat** | **HDM/ALL _SOL NATIVES 13G04nat** | **HDM/ALL_ SOL NATIVES 13G04nat** | **HDM/ALL_SOL NATIVES 13G04nat** |
| **Injected dose (challenge)** | **100µg** | **100µg** | **100µg** | **100µg** | **100µg** | **100µg** | **100µg** | **100µg** |
| **Way of administration (challenge)** | **IP** | **IP** | **IP** | **IP** | **IP** | **IP** | **IP** | **IP** |
| Injected volume **(challenge)** | **200µl** | **200µl** | **200µl** | **200µl** | **200µl** | **200µl** | **200µl** | **200µl** |
| **Injection calendar (challenge)** | **J28** | **J35** | **J42** | **J49** | **J28** | **J35** | **J42** | **J49** |

### 8. Denomination of the groups:

| | |
|---|---|
| Group 1 | 15H20-DP / Challenge J28 |
| Group 2 | 15H20-DP / Challenge J35 |
| Group 3 | 15H20-DP / Challenge J42 |
| Group 4 | 15H20-DP / Challenge J49 |
| Group 5 | PLACEBO (M/T) / Challenge J28 |
| Group 6 | PLACEBO (M/T) / Challenge J35 |
| Group 7 | PLACEBO (M/T) / Challenge J42 |
| Group 8 | PLACEBO (M/T) / Challenge J49 |

### 9. Correspondence between Days post-beginning of the treatment and Days post-Challenge:

All the groups have the same calendar for the treatment phase till the Day 28

| **Groups** | **Days post-beginning of the treatment** | **Days Post-Challenge** |
|---|---|---|
| **1 and 5** | **28-35-42-49-56-63-70-77-84-91** | **0-7-14-21-28-35-42-49-56-63** |
| **2 and 6** | **35-42-49-56-63-70-77-84-91-98** | **0-7-14-21-28-35-42-49-56-63** |
| **3 and 7** | **42-49-56-63-70-77-84-91-98-105** | **0-7-14-21-28-35-42-49-56-63** |
| **4 and 8** | **49-56-63-70-77-84-91-98-105-112** | **0-7-14-21-28-35-42-49-56-63** |

### 10. Results :

Here are the results obtained by ELISA for the production of specific IgG anti-House Dust Mite proteins **(native** - **native proteins coating)**

### a. Evolution of IgG production BEFORE any challenge (from Day 0 to Dav 28):

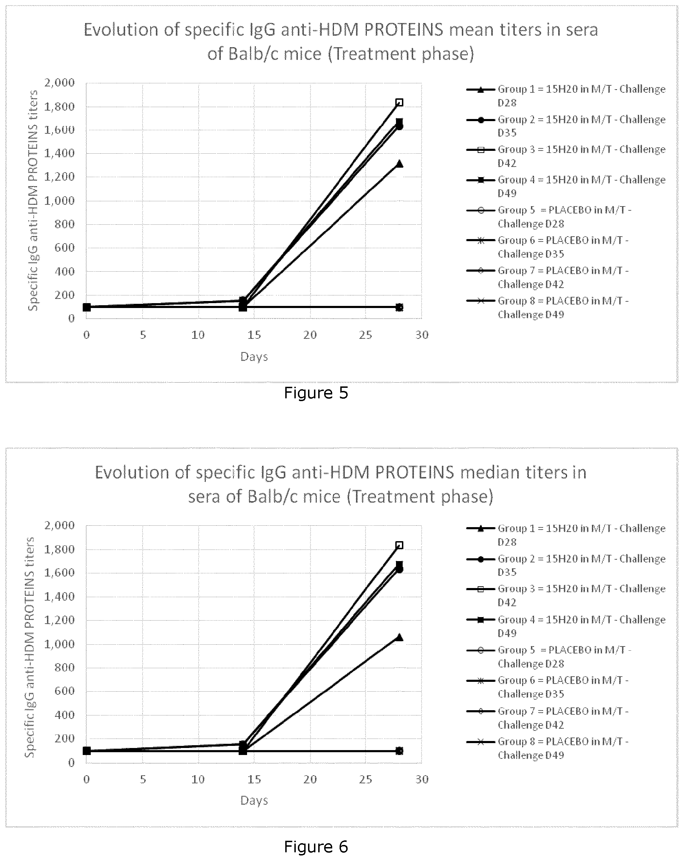

Figures 5 and 6 show that the treatment of the mice with house dust mite peptides as drug product leads to a weak production of specific IgG anti-HDM PROTEINS till the Day 28. The mice treated with PLACEBO show no specific IgG anti-HDM PROTEINS production (Groups 5 to 8 are at the same level)

### b. Evolution of IgG production AFTER the challenge (from the Challenge Dav to the Dav 63 post-challenge):

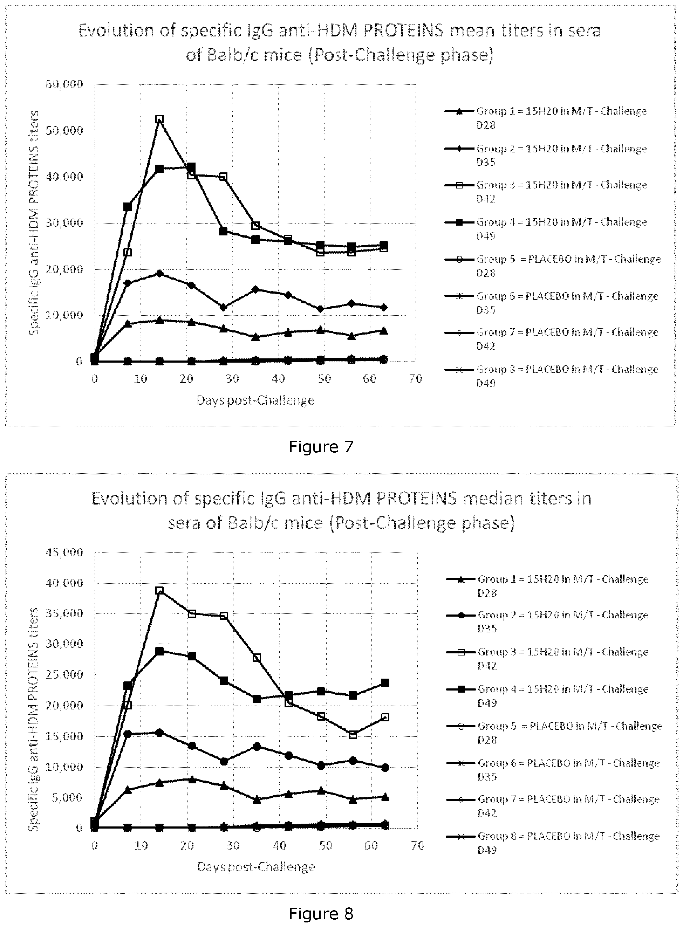

Figures 7 and 8 show the production of specific IgG anti-HDM PROTEINS in the sera of mice belonging to the HDM-peptides treated groups. This production increases to reach a maximum at Day 14 post-Challenge before going down to reach a relative plate state at Day 28 (for Groups 1, 2 and 4) or at Day 35 (for Group 3) post-Challenge. This production (the levels of IgG titers) seems to be linked to the moment when the Challenge occurred: the IgG productions are higher in Groups 3 and 4 (respectively challenged at Day 42 and 49 post-beginning of the treatment) than in Groups 1 and 2 (respectively challenged at Day 28 and 35 post-beginning of the treatment).

The titers values at these specific time-points are the following ones:

| Days Post-Challenge | Specific IgG anti-HDM PROTEINS **MEAN** / **MEDIAN** TITERS | |
|---|---|---|
| | Maximum value Titers | Plate State value Titers |
| Group 1 | D14 = **9.074** / **7.528** | D28 = **7.257** / **6.976** |
| Group 2 | D14 = **19.203** / **15.715** | D28 = **11.813** / **11.040** |
| Group 3 | D14 = **52.547** / **38.826** | D35 = **29.552** / **27.798** |
| Group 4 | D14 = **41.817** / **28.886** | D28 = **28.356** / **24.059** |
| Group 5 | D63 = **458 / 458** | / |
| Group 6 | D56 = **500** / **500** | / |
| Group 7 | D63 = **748** / **748** | / |
| Group 8 | D56 = **462** / **462** | / |

### 11. Conclusions:

From all these results, we can conclude the following points:
- The treatment with HDM-peptides as described in this protocol induces a weak production of specific IgG anti-HDM PROTEINS till Day 28 (post-beginning of the treatment)
- No specific IgG production is observed in Placebo-groups during the treatment phase till Day 28 (post-beginning of the treatment)
- The challenge with a unique dose of 100µg of HDM native proteins potentializes the effect of the treatment allowing an induction of the production of specific IgG anti-HDM PROTEINS reaching a maximum 2 weeks after the challenge. This effect is not observed in the Placebo-groups that received also the same challenge. So, we can conclude that the challenge is not sufficient alone to induce the IgG production (as observed in Placebo-groups) but allows the expression of the HDM-peptides treatment. Obviously, the HDM-peptides treatment can prime the immune system allowing a "memory effect" after a challenge with native proteins.
- The amplitude of the IgG response depends on the moment separating the end of the treatment and the challenge. The response is higher for late-challenged groups.
- All these results are related to HDM native proteins that are the closest proteins to natural allergens.

### Example 3 - Ragweed

Evaluation of the induction of a possible "memory effect" in Balb/c mice treated twice per week during 3 weeks with 100µg or 400µg of ragweed peptides (RAG/PEP_SOL UBT16E03) in mannitol-trehalose environment (the injections were performed sub-cutaneously without any adjuvant) and challenged by a unique ip injection of 100µg of ragweed native proteins without any adjuvant.

### 7. Summary of the protocol:

| | **Groupe 1** | **Groupe 2** | **Groupe 3** | **Groupe 4** | **Groupe 5** | **Groupe 6** | **Groupe 7** |
|---|---|---|---|---|---|---|---|
| **Number of animals** | **10** | **10** | **10** | **10** | **10** | **10** | **10** |
| **Treatment : Antigens in M/T** | **RAG/PEP_SOL 16E03** | **RAG/PEP_SOL 16E03** | **RAG/PEP_SOL 16E03** | **RAG/PEP_SOL 16E03** | **RAG/PEP_SOL 16E03** | **RAG/PEP_SOL 16E03** | **PLACEBO** |
| **Injected dose (treatment)** | **100µg** | **100µg** | **100µg** | **400µg** | **400µg** | **400µg** | **100µg** |
| **Way of administration (treatment)** | **SC** | **SC** | **SC** | **SC** | **SC** | **SC** | **SC** |
| **Injected volume (treatment)** | **200µl** | **200µl** | **200µl** | **200µl** | **200µl** | **200µl** | **200µl** |
| **Injections calendar (treatment)** | **J0-4-7-11-14 and 18** | **J0-4-7-11-14 and 18** | **J0-4-7-11-14 and 18** | **J0-4-7-11-14 and 18** | **J0-4-7-11-14 and 18** | **J0-4-7-11-14 and 18** | **J0-4-7-11-14 and 18** |
| **Challenge** | **RAG/ALL_SOL NAT 16E17** | **RAG/ALL_SOL NAT 16E17** | **RAG/ALL_SOL NAT 16E17** | **RAG/ALL_SOL NAT 16E17** | **RAG/ALL_SOL NAT 16E17** | **RAG/ALL_SOL NAT 16E17** | **RAG/ALL_SOL NAT 16E17** |
| **Injected dose (challenge)** | **100µg** | **100µg** | **100µg** | **100µg** | **100µg** | **100µg** | **100µg** |
| **Way of administration (challenge)** | **IP** | **IP** | **IP** | **IP** | **IP** | **IP** | **IP** |
| **Injected volume (challenge)** | **200µl** | **200µl** | **200µl** | **200µl** | **200µl** | **200µl** | **200µl** |
| **Injection calendar (challenge)** | **J28** | **J42** | **J56** | **J28** | **J42** | **J56** | **J28** |

### 8. Denomination of the groups:

| | |
|---|---|
| Group 1 | 16E03-DP 100 µg / Challenge D28 |
| Group 2 | 16E03-DP 100 µg / Challenge D42 |
| Group 3 | 16E03-DP 100 µg / Challenge D56 |
| Group 4 | 16E03-DP 400 µg / Challenge D28 |
| Group 5 | 16E03-DP 400 µg / Challenge D42 |
| Group 6 | 16E03-DP 400 µg / Challenge D56 |
| Group 7 | PLACEBO (M/T) / Challenge J28 |

### 9. Correspondence between Days post-beginning of the treatment and Days post-Challenge:

All the groups have the same calendar for the treatment phase till the Day 28

| **Groups** | **Days post-beginning of the treatment** | **Days Post-Challenge** |
|---|---|---|
| **1, 4 and 7** | **28-35-42-49-56-63-70-77-84-91** | **0-7-14-21-28-35-42-49-56-63** |
| **2 and 5** | **42-49-56-63-70-77-84-91-98-105** | **0-7-14-21-28-35-42-49-56-63** |
| **3 and 6** | **56-63-70-77-84-91-98-105-112-119** | **0-7-14-21-28-35-42-49-56-63** |

### 10. Results :

Here are the results obtained by ELISA for the production of specific IgG anti-Ragweed proteins **(native** - **native proteins coating)**

### a. Evolution of IgG production BEFORE any challenge (from Day 0 to Dav 28):

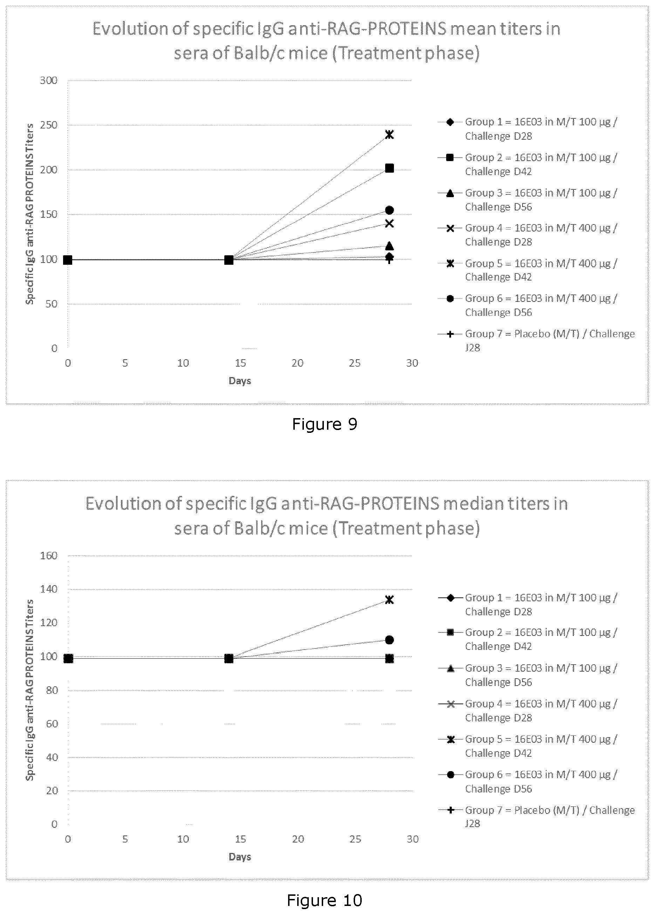

Figures 9 and 10 show that the treatment of mice with both concentration of ragweed peptides as drug product leads to a weak production of specific IgG anti-RAG PROTEINS until Day 28 (Group 1-6). The mice treated with PLACEBO show no production of specific IgG anti-RAG PROTEINS (Group 7)

### b. Evolution of IgG production AFTER the challenge (from the Challenge Dav to Dav 56 post-challenge):

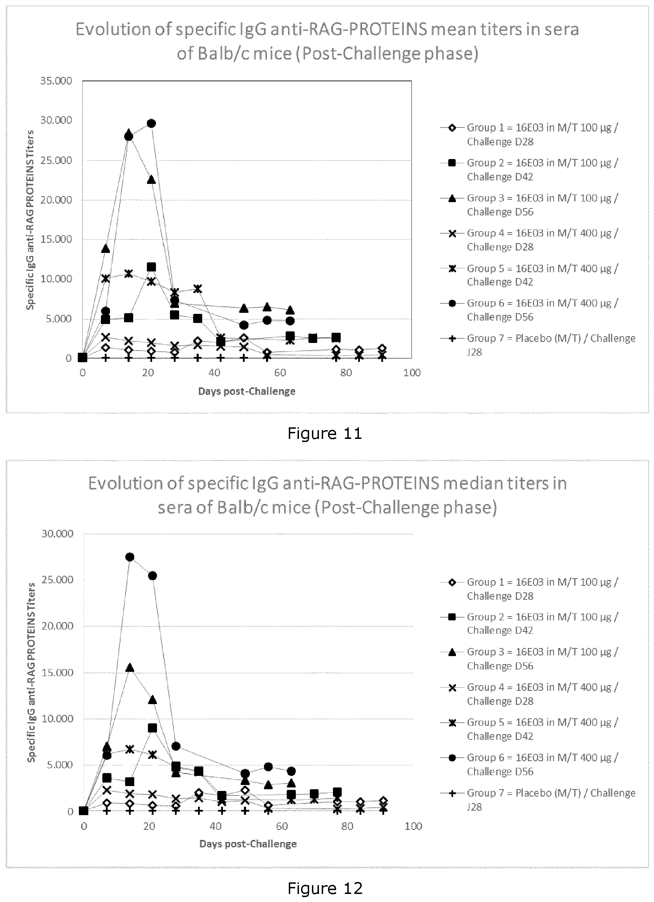

Figures 11 and 12 show the production of specific IgG anti-RAG PROTEINS in the sera of mice belonging to the RAG-peptides treated groups. This production increases to reach a maximum at Day 14-21 post-Challenge before going down to reach a relative plate state at Day 28-42. This production (the levels of IgG titers) seems to be linked to the moment when the Challenge occurred: the IgG productions are higher in Groups 3 and 6 (respectively challenged at Day 56 post-beginning of the treatment) than in Groups 1; 2; 4 and 5 (respectively challenged at Day 28 and Day 42 post-beginning of the treatment).

The titers values at these specific time-points are the following ones:

| Days Post-Challenge | Specific IgG anti-RAG PROTEINS **MEAN** / **MEDIAN** TITERS | |
|---|---|---|
| | Maximum value Titers | Plate State value Titers |
| Group 1 | D49 = **2.604** / **2.319** | D56 = **799** / **722** |
| Group 2 | D21 = **11.569** / **9.041** | D42 = **2.137** / **1.720** |
| Group 3 | D14 = **28.382** / **15.552** | D28 = **6.931** / **4.226** |
| Group 4 | D7 = **2.655** / **2.341** | D56 = **443** / **351** |
| Group 5 | D14 = **10.744** / **6.699** | D42 = **2.620** / **1.361** |
| Group 6 | D21 = 29.673 / **27.507** | D28 = **7.352** / **7.042** |
| Group 7 | D63 = **99** / **99** | D28 = **99** / **99** |

### 11. Conclusions:

From all these results, we can conclude the following points:
- The treatment with RAG-peptides as described in this protocol induces a weak basal production of specific IgG anti-RAG PROTEINS till Day 28 (post-beginning of the treatment)
- No specific IgG production is observed in Placebo-groups during all the study (post-treatment and post-challenge)
- The challenge with a unique dose of 100µg of RAG native proteins potentializes the effect of the treatment allowing an induction of the production of specific IgG anti-RAG PROTEINS reaching a maximum 2 weeks after the challenge. This effect is not observed in the Placebo-groups that received also the same challenge. So, we can conclude that the challenge is not sufficient alone to induce the IgG production (as observed in Placebo-groups) but allows the expression of the RAG-peptides treatment effect. In conclusion, the RAG-peptides treatment can prime the immune system allowing a "memory effect" after a challenge with native proteins.
- The amplitude of the IgG response depends on the delay between the end of the treatment and the challenge. The response is higher for late-challenged groups.
- The amplitude of the IgG response after challenge is dependent of the dose of the treatment with a higher response at 400 µg than at 100 µg when the challenge is performed at Day 28 and Day 42. When the challenge is performed later (Day 56) the IgG responses are similar both dosages, probably due to the amplitude of the response.
- All these results are related to RAG native proteins that are the closest proteins to natural allergens.

## Claims

1. A preparation comprising peptides of a natural allergen wherein the peptides are hydrolyzed allergen peptides for use in the prevention of development of allergy, wherein the preparation is administered prior to the development of any allergy against the allergen used for the preparation.

2. The preparation for use according to claim 1 wherein the preparation is administered 2 to 10 times.

3. The preparation for use according to claim 2 wherein the preparation is administered at intervals of 2 to 10 days.

4. The preparation for use according to claim 2 or 3 wherein the preparation is administered in constant amounts.

5. The preparation for use according to any one of claims 1 to 4 wherein the preparation is free of immune stimulating adjuvants.

6. The preparation for use according to claim 1 wherein said hydrolyzed allergen peptides are obtainable by
a) extracting a natural source of allergens comprising allergenic proteins to form an extract,
b) purifying of said extract to remove non-protein components to form a purified extract,
c) denaturing said purified extract to form a purified denatured extract,
d) hydrolysing the purified denatured extract to form hydrolysed allergen peptides.

7. The preparation for use according to claim 1 wherein said hydrolyzed allergen peptides are obtainable by
a) extracting a source of allergens comprising allergenic proteins to form an extract,
b) purifying the extract to remove non-protein components to form a purified extract,
c) denaturing the purified extract with a first denaturing agent to form a purified denatured extract,
d) refining the purified denatured extract to remove impurities to form a refined denatured extract,
e) denaturing the refined denatured extract with a second denaturing agent to form denatured allergen mixture, and
f) hydrolyzing the denatured allergen mixture to form the hydrolyzed allergen peptides.

8. The preparation for use according to any one of claims 1 to 7 wherein the allergens are selected among pollen allergens, milk allergens, venom allergens, egg allergens, weed allergens, grass allergens, tree allergens, shrub allergens, flower allergens, vegetable allergens, grain allergens, fungi allergens, fruit allergens, berry allergens, nut allergens, seed allergens, bean allergens, fish allergens, shellfish allergens, seafood allergens, meat allergens, spices allergens, insect allergens, mite allergens, mould allergens, animal allergens, pigeon tick allergens, worm allergens, soft coral allergens, animal dander allergens, nematode allergens, allergens of Hevea brasiliensis.

9. The preparation for use of the preceding claims 1 to 8, wherein denaturing is performed with a denaturing agent selected from the group of chaotropic agents, reducing agents and mixtures thereof, preferably among urea, guanidinium chloride, dithiotreitol, thioglycerol, β-mercaptoethanol, TCEP (tris (2-carboxyethyl) phosphine) and mixtures thereof.

10. The preparation for use of one of the preceding claims 1 to 9, wherein the hydrolysis is performed with an enzyme, preferably pepsin, trypsin or chymotrypsin, more preferably wherein hydrolyzing is performed in the presence of a chaotropic agent, preferably selected from urea and guanidinium chloride and reducing reagent preferably from TCEP or DTT.

11. The preparation for use of one of the preceding claims 1 to 10, further comprising purifying the hydrolyzed allergens to remove peptides with molecular weights above 10.000 Da and below 1.000 Da, wherein 70%, more preferably 80% of the peptides are between 10.000 Da and 1.000 Da.

12. The preparation for use according to any one of claims 1 to 11 for use as an allergy vaccine.

## Patentansprüche

1. Präparat, umfassend Peptide eines natürlichen Allergens, wobei die Peptide hydrolysierte Allergenpeptide sind, zur Verwendung bei der Prävention der Entwicklung einer Allergie, wobei das Präparat vor der Entwicklung einer Allergie gegen das für das Präparat verwendete Allergen verabreicht wird.

2. Präparat zur Verwendung gemäß Anspruch 1, wobei das Präparat zwei- bis zehnmal verabreicht wird.

3. Präparat zur Verwendung gemäß Anspruch 2, wobei das Präparat in Abständen von 2 bis 10 Tagen verabreicht wird.

4. Präparat zur Verwendung gemäß Anspruch 2 oder 3, wobei das Präparat in konstanten Mengen verabreicht wird.

5. Präparat zur Verwendung gemäß einem der Ansprüche 1 bis 4, wobei das Präparat frei von immunstimulierenden Adjuvantien ist.

6. Präparat zur Verwendung gemäß Anspruch 1, wobei die hydrolysierten Allergenpeptide erhältlich sind durch
a) Extrahieren einer natürlichen Quelle von Allergenen, die allergene Proteine umfasst, unter Bildung eines Extrakts;
b) Reinigen des Extrakts zur Entfernung von Nichtproteinkomponenten unter Bildung eines gereinigten Extrakts;
c) Denaturieren des gereinigten Extrakts unter Bildung eines denaturierten gereinigten Extrakts;
d) Hydrolysieren des denaturierten gereinigten Extrakts unter Bildung von hydrolysierten Allergenpeptiden.

7. Präparat zur Verwendung gemäß Anspruch 1, wobei die hydrolysierten Allergenpeptide erhältlich sind durch
a) Extrahieren einer Quelle von Allergenen, die allergene Proteine umfasst, unter Bildung eines Extrakts;
b) Reinigen des Extrakts zur Entfernung von Nichtproteinkomponenten unter Bildung eines gereinigten Extrakts;
c) Denaturieren des gereinigten Extrakts mit einem ersten Denaturierungsmittel unter Bildung eines denaturierten gereinigten Extrakts;
d) Raffinieren des denaturierten gereinigten Extrakts zur Entfernung von Verunreinigungen unter Bildung eines raffinierten denaturierten Extrakts;
e) Denaturieren des raffinierten denaturierten Extrakts mit einem zweiten Denaturierungsmittel unter Bildung eines Gemischs von denaturierten Allergenen; und
f) Hydrolysieren des Gemischs von denaturierten Allergenen unter Bildung der hydrolysierten Allergenpeptide.

8. Präparat zur Verwendung gemäß einem der Ansprüche 1 bis 7, wobei die Allergene aus Pollenallergenen, Milchallergenen, Tiergiftallergenen, Eierallergenen, Wildpflanzenallergenen, Grasallergenen, Baumallergenen, Strauchallergenen, Blumenallergenen, Gemüseallergenen, Kornallergenen, Pilzallergenen, Fruchtallergenen, Beerenallergenen, Nussallergenen, Samenallergenen, Bohnenallergenen, Fischallergenen, Schalentierallergenen, Meeresfrüchteallergenen, Fleischallergenen, Gewürzallergenen, Insektenallergenen, Milbenallergenen, Schimmelallergenen, Tierallergenen, Taubenzeckenallergenen, Wurmallergenen, Weichkorallenallergenen, Tierhautschuppenallergenen, Nematodenallergenen, Allergenen von *Hevea brasiliensis* ausgewählt sind.

9. Präparat zur Verwendung gemäß einem der vorstehenden Ansprüche 1 bis 8, wobei das Denaturieren mit einem Denaturierungsmittel durchgeführt wird, das aus der Gruppe der chaotropen Mittel, Reduktionsmittel und Gemische davon, vorzugsweise aus Harnstoff, Guanidiniumchlorid, Dithiothreit, Thioglycerin, β-Mercaptoethanol, TCEP (Tris(2-carboxyethyl)phosphin) und Gemischen davon, ausgewählt ist.

10. Präparat zur Verwendung gemäß einem der vorstehenden Ansprüche 1 bis 9, wobei die Hydrolyse mit einem Enzym, vorzugsweise Pepsin, Trypsin oder Chymotrypsin, durchgeführt wird, wobei besonders bevorzugt das Hydrolysieren in Gegenwart eines chaotropen Mittels, das vorzugsweise aus Harnstoff und Guanidiniumchlorid, und eines Reduktionsmittels, das vorzugsweise aus TCEP oder DTT ausgewählt ist, durchgeführt wird.

11. Präparat zur Verwendung gemäß einem der vorstehenden Ansprüche 1 bis 10, weiterhin umfassend das Reinigen der hydrolysierten Allergene zur Entfernung von Peptiden mit Molekulargewichten oberhalb 10 000 Da und unter 1000 Da, wobei 70%, besonders bevorzugt 80%, der Peptide zwischen 10 000 Da und 1000 Da liegen.

12. Präparat zur Verwendung gemäß einem der vorstehenden Ansprüche 1 bis 11 zur Verwendung als Allergieimpfstoff.

## Revendications

1. Préparation comprenant des peptides d'un allergène naturel dans laquelle les peptides sont des peptides d'allergène hydrolysés pour utilisation dans la prévention du développement d'une allergie, dans laquelle la préparation est administrée avant le développement de toute allergie contre l'allergène utilisé pour la préparation.

2. Préparation pour utilisation selon la revendication 1, dans laquelle la préparation est administrée 2 à 10 fois.

3. Préparation pour utilisation selon la revendication 2, dans laquelle la préparation est administrée à intervalles de 2 à 10 jours.

4. Préparation pour utilisation selon la revendication 2 ou 3, dans laquelle la préparation est administrée en quantités constantes.

5. Préparation pour utilisation selon l'une quelconque des revendications 1 à 4, dans laquelle la préparation est dépourvue d'adjuvants immunostimulants.

6. Préparation pour utilisation selon la revendication 1, dans laquelle lesdits peptides d'allergène hydrolysés peuvent être obtenus par
a) extraction d'une source naturelle d'allergènes comprenant des protéines allergéniques pour former un extrait,
b) purification dudit extrait pour éliminer des composants non-protéine pour former un extrait purifié,
c) dénaturation dudit extrait purifié pour former un extrait dénaturé purifié,
d) hydrolyse de l'extrait dénaturé purifié pour former des peptides d'allergène hydrolysés.

7. Préparation pour utilisation selon la revendication 1, dans laquelle lesdits peptides d'allergène hydrolysés peuvent être obtenus par
a) extraction d'une source d'allergènes comprenant des protéines allergéniques pour former un extrait,
b) purification de l'extrait pour éliminer des composants non-protéine pour former un extrait purifié,
c) dénaturation de l'extrait purifié avec un premier agent dénaturant pour former un extrait dénaturé purifié,
d) affinage de l'extrait dénaturé purifié pour éliminer des impuretés pour former un extrait dénaturé rectifié,
e) dénaturation de l'extrait dénaturé affiné avec un second agent dénaturant pour former un mélange d'allergène dénaturé, et
f) hydrolyse du mélange d'allergène dénaturé pour former les peptides d'allergène hydrolysés.

8. Préparation pour utilisation selon l'une quelconque des revendications 1 à 7, dans laquelle les allergènes sont choisis parmi des allergènes de pollen, des allergènes du lait, des allergènes de venin, des allergènes de l'œuf, des allergènes d'adventices, des allergènes d'herbes, des allergènes d'arbres, des allergènes d'arbustes, des allergènes de fleurs, des allergènes de légumes, des allergènes de céréales, des allergènes de champignons, des allergènes de fruits, des allergènes de baies, des allergènes de fruits à coques, des allergènes de graines, des allergènes de haricots, des allergènes de poisson, des allergènes de coquillages, des allergènes de fruits de mer, des allergènes de viande, des allergènes d'épices, des allergènes d'insectes, des allergènes d'acariens, des allergènes de moisissures, des allergènes d'animaux, des allergènes de tic du pigeon, des allergènes de vers, des allergènes de coraux mous, des allergènes de squames animales, des allergènes de nématodes, des allergènes de Hevea brasiliensis.

9. Préparation pour utilisation selon les revendications 1 à 8 précédentes, dans laquelle la dénaturation est réalisée avec un agent dénaturant choisi dans le groupe des agents chaotropiques, des agents réducteurs et de mélanges de ceux-ci, de préférence parmi l'urée, le chlorure de guanidinium, le dithiotréitol, le thioglycérol, le β-mercaptoéthanol, la TCEP (tris(2-carboxyéthyl)phosphine) et des mélanges de ceux-ci.

10. Préparation pour utilisation selon l'une des revendications 1 à 9 précédentes, dans laquelle l'hydrolyse est réalisée avec une enzyme, de préférence la pepsine, la tripsine ou la chymotrypsine, de manière davantage préférée dans laquelle l'hydrolyse est réalisée en présence d'un agent chaotropique, choisi de préférence parmi l'urée et le chlorure de guanidinium et un agent réducteur de préférence parmi la TCEP ou le DTT.

11. Préparation pour utilisation selon l'une quelconque des revendications 1 à 10 précédentes, comprenant en outre la purification des allergènes hydrolysés pour éliminer des peptides avec des poids moléculaires supérieurs à 10 000 Da et inférieurs à 1 000 Da, dans laquelle 70 %, de manière davantage préférée 80 % des peptides sont entre 10 000 Da et 1 000 Da.

12. Préparation pour utilisation selon l'une quelconque des revendications 1 à 11, pour utilisation en tant que vaccin contre une allergie.
